# EUROPEAN PATENT SPECIFICATION

(11) **EP 4 172 367 B1**
(45) Date of publication and mention of the grant of the patent: **15.01.2025**
(21) Application number: 21737038.6
(22) Date of filing: 25.06.2021
(51) Int. Cl.: C12Q 1/6883

(54) **A METHOD OF PREDICTING RESPONSE TO TREATMENT WITH A DISEASE-MODIFYING ANTI-RHEUMATIC DRUG, AND/OR CLASSIFYING DISEASE ACTIVITY IN A SUBJECT WITH RHEUMATOID ARTHRITIS**
VERFAHREN ZUR VORHERSAGE DER REAKTION AUF EINE BEHANDLUNG MIT EINEM KRANKHEITSMODIFIZIERENDEN ANTIRHEUMATIKUM UND/ODER ZUR KLASSIFIZIERUNG DER KRANKHEITSAKTIVITÄT BEI RHEUMATOIDER ARTHRITIS
PROCÉDÉ DE PRÉDICTION DE LA RÉPONSE AU TRAITEMENT PAR UN MÉDICAMENT ANTIRHUMATISMAL MODIFICATEUR DE LA MALADIE, ET/OU DE CLASSIFICATION DE L'ACTIVITÉ DE LA MALADIE CHEZ UN SUJET ATTEINT DE POLYARTHRITE RHUMATOÏDE

(30) Priority: 25.06.2020 GB 202009689
(43) Date of publication of application: 03.05.2023
(73) Proprietor: University Of Ulster, County Londonderry BT52 1SA (GB)
(72) Inventor: GIBSON, David, Londonderry BT52 1SA (GB); BJOURSON, Tony, Londonderry BT52 1SA (GB); EAKIN, Amanda, Londonderry BT52 1SA (GB); GARDINER, Philip, Londonderry BT47 6SB (GB); WRIGHT, Gary, Belfast City Hospital Lisburn Road BT9 7AB (GB); TODD, Naomi, Londonderry BT52 1SA (GB)
(74) Representative: FRKelly
(86) International application number: PCT/EP2021/067497
(87) International publication number: WO 2021/260180

(56) References cited:
- WO-A1-2019/147779
- US-B2- 7 935 482
- SEIJI NAKAMURA ET AL: "Identification of baseline gene expression signatures predicting therapeutic responses to three biologic agents in rheumatoid arthritis: a retrospective observational study", ARTHRITIS RESEARCH & THERAPY, vol. 18, no. 1, 19 July 2016 (2016-07-19), XP055465500, DOI: 10.1186/s13075-016-1052-8
- JING CUI ET AL: "Genome-Wide Association Study and Gene Expression Analysis Identifies CD84 as a Predictor of Response to Etanercept Therapy in Rheumatoid Arthritis", PLOS GENETICS, vol. 9, no. 3, 28 March 2013 (2013-03-28), pages e1003394 - 1, XP055290570, DOI: 10.1371/journal.pgen.1003394
- ANUP SINGH ET AL: "Study of correlation of level of expression of Wnt signaling pathway inhibitors sclerostin and dickkopf-1 with disease activity and severity in rheumatoid arthritis patients", DD&T DRUG DISCOVERIES & THERAPEUTICS, vol. 13, no. 1, 28 February 2019 (2019-02-28), Japan, pages 22 - 27, XP055770894, ISSN: 1881-7831, DOI: 10.5582/ddt.2019.01011
- DIARRA DANIELLE ET AL: "Dickkopf-1 is a master regulator of joint remodeling", NATURE MEDICINE, NATURE PUB. CO, NEW YORK, vol. 13, no. 2, 1 February 2007 (2007-02-01), pages 156 - 163, XP002486201, ISSN: 1078-8956, [retrieved on 20070121], DOI: 10.1038/NM1538
- MARSHALL M J ET AL: "Increased circulating Dickkopf-1 in Paget's disease of bone", CLINICAL BIOCHEMISTRY, ELSEVIER INC, US, CA, vol. 42, no. 10-11, 1 July 2009 (2009-07-01), pages 965 - 969, XP026173702, ISSN: 0009-9120, [retrieved on 20090421], DOI: 10.1016/J.CLINBIOCHEM.2009.04.007

## Description

### Field of the Invention

The present invention relates to a method of predicting response to treatment with a disease-modifying anti-rheumatic drug, and/or classifying disease activity in a subject with rheumatoid arthritis. The method comprises determining the quantitative level of one or more biomarkers in a biological sample from the subject and either predicting response to treatment with a disease-modifying anti-rheumatic drug, classifying disease activity, and/or predicting response to treatment with a disease-modifying anti-rheumatic drug and classifying disease activity; based on the quantitative level of the or each biomarker in the biological sample.

### Background to the Invention

Many studies have highlighted the need for robust biomarkers in rheumatoid arthritis (RA) in order to assist with diagnosis, prognosis and determining treatment response. The human plasma proteome is of particular interest as it contains proteins secreted or shed by circulating cells and tissue involved in the pathogenesis of RA. When the body is under stress, for example during chronic inflammation, the plasma proteome changes as part of the immune response. This study focusses on identifying and quantifying changes in circulating plasma proteins between patients with variable treatment responses, as this approach can also improve our understanding of disease pathogenesis.

Disease-modifying anti-rheumatic drugs (DMARDs) are immunosuppressive and immunomodulatory agents, and are classified as either conventional DMARDs (cDMARDs) or biologic DMARDs (bDMARDs). Commonly used conventional DMARDs include methotrexate, leflunomide, hydroxychloroquine, and sulfasalazine. Biologic DMARDs were introduced in the early 1990s and are usually prescribed after the failure of conventional DMARD therapy (ongoing disease activity, or clinical or radiographic disease progression). Some biologic agents include infliximab, adalimumab, etanercept, rituximab, abatacept, rituximab, tocilizumab, tofacitinib, among others. Biologic DMARDs are highly specific and target a specific pathway of the immune system.

Currently, clinicians consider C-reactive protein (CRP) a biomarker of inflammation. Although NICE guidelines recommend regular monitoring of CRP levels throughout treatment of RA (NICE 2009), some studies suggest it can be used as a substitute of erythrocyte sedimentation rate (ESR) in composite scores used to determine treatment response such as the Disease Activity Score in 28 joints (DAS28). However, the increase in plasma CRP levels observed in RA compared to health is caused in part by the secretion of IL-6 from T-cells and macrophages during inflammation and conflicting evidence brings into question CRP's reliability as a marker of joint inflammation or disease activity. The lack of specificity of CRP to RA pathology may be a contributing factor to this contradictory evidence.

The wide variety of proteomic techniques now available has enabled research groups to robustly analyse a number of biological sample types. The most obvious, pathologically proximal sample to study disease activity in RA patients is synovial fluid. For example, a study using isobaric tag labelling of synovial peptides identified over 500 proteins that were differentially expressed between osteoarthritis (OA) and RA patients.

Cytosolic proteins isolated from synovial tissue and analysed by 2-dimensional gel electrophoresis discovered protein expression patterns which differentiate RA from spondyloarthropathy, enabling earlier diagnosis. Similar studies have used the same method to analyse proteins from fibroblast-like synoviocyte (FLS) cells and have also identified potential novel diagnostic proteins for RA. The analysis of proteins from synovial fluid or tissue may be useful in understanding disease pathogenesis, as this is the target of the autoimmune response in RA. However, this sampling method is rather invasive, and it is an impractical sample in comparator healthy control groups. Although the study of proteins from tissue-specific cells is advantageous in understanding the cell biology of RA, the lengthy laboratory procedures are somewhat unrealistic for regular determination of treatment response.

An increasing number of studies have investigated potential circulating biomarkers of treatment response in RA. Some studies have looked at circulating cellular markers of response. Peripheral blood is a potentially rich source of biomarker candidates to determine treatment response in RA. However, previous studies have failed to identify robust proteins that are indicative of both disease activity and treatment response. This study focuses on the circulating plasma proteome in RA as an accessible source of potential response biomarkers.

WO 2019/147779 relates to diagnostic methods, therapeutic methods, and kits for treating an individual having rheumatoid arthritis. US7935482 relates to the use of gene expression data in the identification, monitoring and treatment of rheumatoid arthritis and in characterization and evaluation of inflammatory conditions induced or related to rheumatoid arthritis. SEIJI NAKAMURA ET AL (2016-07-19) identified original gene expression predictive signatures uniquely underlying the therapeutic effects of DMARDs: IFX, TCZ, and ABT. JING CUI ET AL (2013-03-28) suggests that CD84 genotype and/or expression may prove to be a biomarker for etanercept response in RA patients. ANUP SINGH ET AL (2019-02-28), DIARRA DANIELLE ET AL (2007-01-21) and MARSHALL M J ET AL (2009-04-21) show that serum levels of Dkk-1 in rheumatoid arthritis patients were shown to be proportional to disease activity.

### Summary of the Invention

According to a first aspect of the present invention, there is provided an *in vitro* method of predicting response to treatment with a disease-modifying anti-rheumatic drug (DMARD), and/or classifying disease activity in a subject with rheumatoid arthritis in a subject, the method comprising the steps of:
(a) determining the quantitative level of two or more biomarkers in a biological sample from the subject; and
(b) predicting response to treatment with a DMARD. and/or classifying disease activity in a subject with rheumatoid arthritis in the subject based on the quantitative level of the or each biomarker in the biological sample;
wherein the biomarker is DECR1 and CD40-L.

Optionally, the two biomarkers for predicting response to treatment with a DMARD, and/or classifying disease activity further comprises ITGB1BP2.

Optionally, the two biomarkers for predicting response to treatment with a DMARD, and/or classifying disease activity further comprises CASP-3.

Optionally, the two biomarkers for predicting response to treatment with a DMARD, and/or classifying disease activity further comprises PDFG subunit A.

Optionally, the two biomarkers for predicting response to treatment with a DMARD, and/or classifying disease activity further comprises Dkk-1.

Optionally, the two biomarkers for predicting response to treatment with a DMARD, and/or classifying disease activity further comprises SELP.

Optionally, the two biomarkers for predicting response to treatment with a DMARD, and/or classifying disease activity further comprises SOD2.

Optionally, the two biomarkers for predicting response to treatment with a DMARD, and/or classifying disease activity further comprises SORT1.

Optionally, the two biomarkers for predicting response to treatment with a DMARD, and/or classifying disease activity further comprises CD84.

Optionally, there is provided a method of predicting response to treatment with a disease-modifying anti-rheumatic drug (DMARD), and/or classifying disease activity in a subject with rheumatoid arthritis, the method comprising the steps of:
(a) determining the quantitative level of two or more biomarkers in a biological sample from the subject; and
(b) predicting response to treatment with a DMARD, and/or classifying disease activity based on the quantitative level of the two biomarkers in the biological sample;
wherein the two biomarkers are DECR1 and CD40-L, further comprising any one or more of ITGB1BP2, CASP-3, PDFG subunit A, Dkk-1, SELP, SOD2, SORT1 and CD84.

Optionally, the two or more biomarkers is a gene.

Optionally, the two or more biomarkers is a nucleic acid.

Optionally, the two or more biomarkers is a deoxyribonucleic acid.

Optionally, the two or more biomarkers is a ribonucleic acid.

Preferably, the two or more biomarkers is a protein.

Optionally, the two or more biomarkers is a peptide.

Optionally, the two or more biomarkers for predicting response to treatment with a DMARD, and/or classifying disease activity is a gene having a GenBank Accession Version Number selected from one or more of AK300069.1, L07414.1, BC108901.2, AJ413269.1, AH002928.2, AH009834.2, AL022146.1, X07834.1, AL390252.9 and AF054816.1.

Preferably, the two or more biomarkers for predicting response to treatment with a DMARD, and/or classifying disease activity is a protein having a UniProt ID selected from one or more of Q16698, P29965, Q9UKP3, P42574, P04085, O94907, P16109, P04179, Q99523 and Q9UIB8.

Optionally, the two or more biomarkers for predicting response to treatment with a DMARD further comprises CASP-3, SELP, JAM-A, PDFG subunit A, ITGB1BP2, Dkk-1, PECAM-1, ANG-1, SOD2, DAPP1, DCTN1, PAI and CD40-L.

Optionally, the two or more biomarkers for predicting response to treatment with a DMARD is selected from one or more of DECR1, CASP-3, SELP, JAM-A, PDGF subunit A, ITGB1BP2, Dkk-1, PECAM-1, ANG-1, SOD2, DAPP1, DCTN1, PAI and CD40-L.

Optionally, the two or more biomarkers for predicting response to treatment with a DMARD further comprises HB-EGF, HCLS1, IRAK4, STK4, SH2B3, NEMO, CD84, EIF4G1, PRDX1, TANK, CXCL1, SIRT2, ZBTB16, NF2, PDGF subunit B, SPRY2, HEXIM1, PPP1R9B, PRDX3, PAR-1, ICA1, CCL17, STAMPB, MGMT, SRPK2, BML hydrolase, PLXNA4 and SORT1.

Optionally, the two or more biomarkers for predicting response to treatment with a DMARD is selected from one or more of DECR1, CASP-3, SELP, JAM-A, PDGF subunit A, ITGB1BP2, Dkk-1, PECAM-1, ANG-1, SOD2, DAPP1, DCTN1, PAI, CD40-L, HB-EGF, HCLS1, IRAK4, STK4, SH2B3, NEMO, CD84, EIF4G1, PRDX1, TANK, CXCL1, SIRT2, ZBTB16, NF2, PDGF subunit B, SPRY2, HEXIM1, PPP1R9B, PRDX3, PAR-1, ICA1, CCL17, STAMPB, MGMT, SRPK2, BML hydrolase, PLXNA4 and SORT1.

Optionally, the two or more biomarkers for predicting response to treatment with a DMARD is a gene having a GenBank Accession Version Number selected from one or more of AK300069.1, AJ413269.1, AL022146.1, AL136649.1, AH002928.2, BC108901.2, BC107046.2, JQ287500.1, AY124380.1, X07834.1, AF178987.1 AK314352.1, X04731.1, AK222896.1, AC004634.1, AK312750.1, AY340965.1, Z93016.2, BC136451.1, AJ271718.1, AF054816.1, AK131407.1, CR407652.1, AC009299.5, BT006880.1, KF032391.1, BC029812.1, Y18000.1, M12783.1, AL354668.13, BC006460.1, KF495721.1, BT020007.1, BC002464.2, U37183.1, CH471092.1, CH471053.2, M60761.1, AC004884.1, AK312896.1, AC009365.9 and AL390252.9.

Preferably, the two or more biomarkers for predicting response to treatment with a DMARD is a protein having a UniProt ID selected from one or more of Q16698, P42574, P16109, Q9Y624, P04085, Q9UKP3, Q9UBT3, P16284, Q15389, P04179, Q9UN19, Q14203, P05121, P25942, Q99075, P14317, Q9NWZ3, Q13043, Q9UQQ2, Q9Y6K9, Q9UIB8, Q04637, Q06830, Q92844, P09341, Q8IXJ6, Q05516, P35240, P01127, O43597, O94992, Q96SB3, P30048, P25116, Q05084, Q92583, 095630, P16455, P78362, Q13867, Q9HCM2 and Q99523.

Preferably, the two or more biomarkers for predicting treatment response in a subject treated with a DMARD is selected from one or more of DECR1, CD40-L, ITGB1BP2, CASP-3, PDFG subunit A, Dkk-1, SELP, SOD2, SORT1 and CD84.

Optionally, there is provided a method of classifying disease activity in a subject with rheumatoid arthritis, the method comprising the steps of:
(a) determining the quantitative level of two or more biomarkers in a biological sample from the subject; and
(b) classifying disease activity based on the quantitative level of two or more biomarkers in the biological sample;
wherein the biomarkers are selected from DECR1 and CD40-L.

Optionally, the two or more biomarkers for classifying disease activity further comprises CD40-L, ITGB1BP2, CASP-3, PDFG subunit A, Dkk-1, SELP, SOD2, SORT1 and CD84.

Optionally, the two or more biomarkers for classifying disease activity is a gene having a GenBank Accession Version Number selected from one or more of AK300069.1, L07414.1, BC108901.2, AJ413269.1, AH002928.2, AH009834.2, AL022146.1, X07834.1, AL390252.9 and AF054816.1 .

Preferably, the two or more biomarkers for classifying disease activity is a protein having a UniProt ID selected from one or more of Q16698, P29965, Q9UKP3, P42574, P04085, O94907, P16109, P04179, Q99523 and Q9UIB8 .

Further optionally, the determining step (a) comprises determining the quantitative level of three, four, five, six, seven, eight, nine, ten, eleven, twelve, thirteen, fourteen, fifteen, twenty, twenty five, thirty, thirty five, forty, forty two or more biomarkers in the biological sample from the subject.

Optionally, the determining step (a) comprises determining the quantitative level of all of the biomarkers in the biological sample from the subject.

Optionally or additionally, the determining step (a) comprises determining the quantitative level of each of the biomarkers in the biological sample from the subject.

Optionally, the biological sample is selected from whole blood, serum, plasma, urine, interstitial fluid, peritoneal fluid, cervical swab, tears, saliva, buccal swab, skin, brain tissue, and cerebrospinal fluid.

Further optionally, the biological sample is selected from whole blood, serum, and plasma.

Preferably, the biological sample is whole blood.

Further preferably, the biological sample is plasma.

Optionally, the predicting and/or classifying step (b) comprises comparing the quantitative level of two or more biomarkers in the biological sample from the subject with the quantitative level of the or each respective biomarkers in a normal sample.

Optionally, the normal sample is a biological sample from a subject not treated with a DMARD.

Optionally, the normal sample is a biological sample from a subject not treated with a DMARD and not suffering from rheumatoid arthritis.

Optionally, the subject is treated with one or more DMARDs

Optionally, the subject is treated with one or more DMARDs, including any one or more of infliximab, adalimumab, etanercept, rituximab, abatacept, rituximab, tocilizumab, certolizumab, golimumab, anakinra, baricitinib, upadacitinab and tofacitinib.

Optionally, the subject is treated with one or more DMARDs, including any one or more of infliximab, adalimumab, etanercept, rituximab, abatacept, rituximab, tocilizumab, certolizumab, golimumab, anakinra, baricitinib, upadacitinab and tofacitinib, and will respond to treatment (responder). Optionally, in the method of predicting response to treatment with a DMARD, the subject is treated with one or more DMARDs including any one or more of infliximab, adalimumab, etanercept, rituximab, abatacept, rituximab, tocilizumab, certolizumab, golimumab, anakinra, baricitinib, upadacitinab and tofacitinib, and will not respond to treatment (non-responder).

Optionally, the method is a method of predicting response to treatment with a conventional disease-modifying anti-rheumatic drug (cDMARD).

Optionally, the subject is treated with one or more cDMARDs.

Optionally, the subject is treated with one or more cDMARDs, including any one or more of apremilast, azathioprine, ciclosporin, cyclophosphamide, sodium aurothiomalate, mycophenolate mofetil, penicillamine, tacrolimus,, methotrexate, sulfasalazine, leflunomide and hydroxychloroquine.

Optionally, the subject is treated with one or more cDMARDs, including any one or more of apremilast, azathioprine, ciclosporin, cyclophosphamide, sodium aurothiomalate, mycophenolate mofetil, penicillamine, tacrolimus, methotrexate, sulfasalazine, leflunomide and hydroxychloroquine, and will respond to treatment (responder).

Optionally, in the method of predicting response to treatment with a cDMARD, the subject is treated with one or more cDMARDs including apremilast, azathioprine, ciclosporin, cyclophosphamide, sodium aurothiomalate, mycophenolate mofetil, penicillamine, tacrolimus, methotrexate, sulfasalazine, leflunomide and hydroxychloroquine, and will not respond to treatment (non-responder).

Optionally, the determining step (a) further comprises determining the quantitative level in a first set of two or more respective biomarkers.

Optionally, the determining step (a) further comprises determining the quantitative level in a first set of respective biomarkers(s) wherein the quantitative level of the first set of biomarkers is greater than the quantitative level of the respective biomarkers(s) in a normal sample, which is indicative of a non-responder.

Optionally, the first set of biomarkers is selected from any one or more of DECR1, CASP-3, SELP, JAM-A, PDFG subunit A, ITGB1BP2, Dkk-1, PECAM-1, ANG-1, SOD2, DAPP1, DCTN1, PAI, CD40-L, HB-EGF, HCLS1, IRAK4, STK4, SH2B3, NEMO, CD84, EIF4G1, PRDX1, TANK, CXCL1, SIRT2, ZBTB16, NF2, PDGF subunit B, SPRY2, HEXIM1, PPP1R9B, PRDX3, PAR-1, ICA1, CCL17, STAMPB, MGMT, SRPK2, BML hydrolase, PLXNA4 and SORT1.

Optionally, the determining step (a) further comprises determining the quantitative level in a second set of respective biomarkers(s).

Optionally, the determining step (a) further comprises determining the quantitative level in a second set of respective biomarkers(s) wherein the quantitative level of the second set of biomarkers is less than the quantitative level of the respective biomarkers(s) in a normal sample, which is indicative of a non-responder.

Optionally, the second set of biomarkers is selected from any one or more of DECR1, CD40-L, ITGB1BP2, CASP-3, PDGF subunit A, Dkk-1, SELP, SOD2, SORT1 and CD84.

Further optionally, the quantitative level of the or each biomarker is higher than a threshold value and is indicative of a non-responder, wherein the respective threshold value of the or each biomarker is:

| **Biomarker** | **Threshold value** |
|---|---|
| DECR1 | 7.1320 |
| CASP-3 | 10.4740 |
| SELP | 10.5279 |
| PDGF subunit A | 5.5889 |
| ITGB1BP2 | 6.2200 |
| Dkk-1 | 10.4467 |
| SOD2 | 9.9025 |
| CD40-L | 7.4976 |
| CD84 | 6.0718 |
| SORT1 | 8.5585 |

Optionally, the quantitative level of the or each biomarker is lower than a threshold value and is indicative of a responder, wherein the respective threshold value of the or each biomarker is:

| **Biomarker** | **Threshold value** |
|---|---|
| DECR1 | 7.1320 |
| CASP-3 | 10.4740 |
| SELP | 10.5279 |
| PDGF subunit A | 5.5889 |
| ITGB1BP2 | 6.2200 |
| Dkk-1 | 10.4467 |
| SOD2 | 9.9025 |
| CD40-L | 7.4976 |
| CD84 | 6.0718 |
| SORT1 | 8.5585 |

Optionally, the quantitative level of the or each biomarker is between two threshold values and is indicative of high disease activity, wherein the respective threshold values of the or each biomarker are:

| **Biomarker** | **Threshold value** |
|---|---|
| DECR1 | 7.8984 - 11.6893 |
| CD40-L | 8.4728 - 12.7503 |
| ITGB1BP2 | 6.7802 - 11.4759 |
| CASP-3 | 11.4114 - 13.6768 |
| PDFG subunit A | 5.7334 - 8.4577 |
| Dkk-1 | 11.2034 - 12.7568 |
| SELP | 11.3715 - 12.6136 |
| SOD2 | 10.0471 - 10.5467 |
| SORT1 | 8.7990 - 9.7006 |
| CD84 | 6.1830 - 7.8683 |

Optionally, the quantitative level of the or each biomarker is between two threshold values and is indicative of moderate disease activity, wherein the respective threshold values of the or each biomarker are:

| **Biomarker** | **Threshold values** |
|---|---|
| DECR1 | 5.6510 - 10.5039 |
| CD40-L | 6.1622 - 10.1159 |
| ITGB1BP2 | 3.1210 - 8.5301 |
| CASP-3 | 8.0945 - 12.2662 |
| PDFG subunit A | 3.9517 - 7.0510 |
| Dkk-1 | 9.1861 - 12.0037 |
| SELP | 9.2287 - 11.9099 |
| SOD2 | 9.5039 - 10.2607 |
| SORT1 | 7.5225 - 9.2595 |
| CD84 | 4.9694 - 6.8399 |

Optionally, the quantitative level of the or each biomarker is between two threshold values and is indicative of low disease activity, wherein the respective threshold values of the or each biomarker are:

| **Biomarker** | **Threshold values** |
|---|---|
| DECR1 | 6.6490 - 6.7309 |
| CD40-L | 6.5871 - 6.9401 |
| ITGB1BP2 | 4.1040 - 4.5746 |
| CASP-3 | 8.3368 - 8.7173 |
| PDFG subunit A | 3.8283 - 4.0907 |
| Dkk-1 | 9.6015 - 9.8410 |
| SELP | 8.2174 - 9.3414 |
| SOD2 | 9.6542 - 9.9702 |
| SORT1 | 7.7980 - 8.5272 |
| CD84 | 5.2689 - 5.5881 |

Optionally, the quantitative level of the or each biomarker is between two threshold values and is indicative of in remission, wherein the respective threshold values of the or each biomarker are:

| **Biomarker** | **Threshold value** |
|---|---|
| DECR1 | 5.6064 - 6.9173 |
| CD40-L | 6.2510 - 7.3891 |
| ITGB1BP2 | 3.4508 - 5.5040 |
| CASP-3 | 7.9543 - 9.2353 |
| PDFG subunit A | 2.7648 - 4.9645 |
| Dkk-1 | 9.2598 - 9.9684 |
| SELP | 8.8272 - 9.9551 |
| SOD2 | 9.5195 - 9.7582 |
| SORT1 | 7.6829 - 8.4218 |
| CD84 | 4.6188 - 5.8750 |

Optionally, the method is a method of classifying disease activity, wherein the subject has high disease activity, moderate disease activity, low disease activity or is in remission.

Optionally, in a subject with high disease activity, the quantitative level of the or each biomarker is higher compared to a subject with moderate disease activity, low disease activity and in remission.

Optionally, in a subject with moderate disease activity, the quantitative level of the or each biomarker is higher compared to a subject with low disease activity and in remission, but the quantitative level of the or each biomarker is lower compared to a subject with high disease activity.

Optionally, in a subject with low disease activity, the quantitative level of the or each biomarker is higher compared to a subject with in remission, but the quantitative level of the or each biomarker is lower compared to a subject with high disease activity and moderate disease activity.

Optionally, in a subject in remission, the quantitative level of the or each biomarker is lower compared to a subject with high disease activity, moderate disease activity and low disease activity.

Optionally, the method of classifying disease activity is a method of prognosing disease activity.

Optionally, the method of predicting response to treatment with a DMARD, and/or classifying disease activity is an *in vitro* method.

Optionally, the method of predicting response to treatment with a cDMARD, and/or classifying disease activity is an *in vitro* method.

Optionally, the method of predicting response to treatment with a DMARD is an *in vitro* method.

Optionally, the method of predicting response to treatment with a cDMARD is an *in vitro* method.

Optionally, the method of classifying disease activity is an *in vitro* method.

Optionally, the method of prognosing disease activity is an *in vitro* method.

Also disclosed but not part of the invention is a method of treating rheumatoid arthritis by predicting response to treatment with a DMARD; and treating the subject.

Also disclosed but not part of the invention is method of treating rheumatoid arthritis by predicting response to treatment with a cDMARD; and treating the subject.

Also disclosed but not part of the invention is method of treating rheumatoid arthritis by classifying disease activity; and treating the subject.

Also disclosed but not part of the invention is method of treating rheumatoid arthritis by predicting response to treatment with a DMARD, and/or classifying disease activity; and treating the subject.

Also disclosed but not part of the invention is method of treating rheumatoid arthritis by predicting response to treatment with a cDMARD, and/or classifying disease activity; and treating the subject.

### Brief Description of the Drawings

Embodiments of the present invention will now be described with reference to the accompanying drawings and following non-limiting examples, in which:
**Figure 1** illustrates unsupervised clustering of proteins calculated to be significantly different between DMARD naïve (normal) (N), DMARD responder (DR) and DMARD non-responder (DNR) RA patients;
**Figure 2** illustrates unsupervised clustering of proteins calculated to be significantly different between RA patients with high, moderate or low disease activity, or who are in remission. Disease activity classifications are based on EULAR guidelines;
**Figure 3** illustrates ROC curve analysis of each protein when a cut-off (y-intercept) value is applied to distinguish RA cDMARD responders and non-responders; and
**Figure 4** illustrates correlation of NPX values of top ten proteins of interest with DAS28-ESR score.

### Examples

### Materials & Methods

The research team at Ulster University collaborated with rheumatologists from the Western Health and Social Care Trust and the Belfast Health and Social Care Trust to design, conduct and recruit patients to the study. Informed consent to participate was obtained from all RA patients enrolled in the study (Table 1).

**Table. 1. Demographics of patients treated with conventional disease-modifying anti-rheumatic drugs DN = DMARD naïve (normal), DR = DMARD responder, DNR = DMARD non-responder.**

| | DN (n=6) | DR (n=24) | DNR (n=32) |
|---|---|---|---|
| Female, *n* (%) | 5 (83.3) | 22 (91. 7) | 27 (81.8) |
| Age, mean (SD), years | 42.5 (16.7) | 59.7(12.2) | 57.7(10.1) |
| Treatment duration since diagnosis, mean (SD), years | N/A | 5.0 (3.8) | 10.8 (12.1) |
| Erythrocyte Sedimentation Rate (ESR), mean (SD), mm in Ihr | 23.5 (15.2), n=6 | 12.2 (7.9), n=24 | 24.1 (20.0), n=29 |
| C-reactive Protein (CRP), mean (SD), mg/L | 6.1 (2.9), n=6 | 5.3 (7.3), n=22 | 10.6 (9.4), n=28 |
| Disease Activity Score in 28 joints (DAS28-ESR), mean (SD) | 4.3 (0.4), n=4 | 2.6 (1.0), n=24 | 4.9 (1.1), n=19 |

Diagnosis for all patients fulfilled the 2010 American College of Rheumatology (ACR) criteria (American College of Rheumatology and European League Against Rheumatism 2010). Patients were treated with one or more conventional disease-modifying anti-rheumatic drugs (cDMARD), including methotrexate, sulfasalazine, leflunomide and hydroxychloroquine.

Patients were classified as 'responders' or 'non-responders' according to the National Institute for Health and Clinical Excellence (NICE) guidelines, where responders have exhibited a change in DAS28-ESR of >1.2 following treatment (NICE 2009). Responders and non-responders had undergone treatment for an average of 5 and 11 years respectively.

Disease activity classifications were according to the European League Against Rheumatism (EULAR) criteria, where a DAS28-ESR score of ≥5.1 was classified as 'high' disease activity (n=9), >3.2 and ≤5.1 was 'moderate' (n=21), >2.6 and ≤3.2 was 'low' (n=2), and <2.6 was classified as 'remission' (n=11).

Office for Research Ethics Committees Northern Ireland (ORECNI) (11/NI/0188) and Ulster University Research Ethics Committee (UREC) (REC/14/0053) approvals were obtained for the study.

### Blood processing

Peripheral whole blood used for plasma extraction was collected in an EDTA coated tube (Aquilant Scientific) using a venepuncture technique. Blood tubes were stored at 4 °C for a maximum of 2 hours until processing. The blood tube was centrifuged in an Eppendorf 5804 centrifuge at 300 rcf for 10 minutes at 18 °C. The majority of the upper plasma layer was removed and stored at -80 °C for later analysis.

### Proximity Extension Assay (PEA)

Plasma from each patient sample was thawed and a small volume of ~40 µl was sent to OLINK (Analysis service Uppsala, Sweden). Each sample was analysed across OLINK's CVD II, CVD III, immune response and inflammatory panels. Each high throughput multiplex immunoassay consisted of 92 proteins.

The technology used is known as 'Proximity Extension Assay' (PEA), where matched antibody pairs containing unique DNA sequences were incubated with the sample. Sequences that were exactly matched hybridized and were extended using DNA polymerase. The elongated DNA was then amplified and a qPCR readout using Fluidigm^{®} BioMARKTM was used to determine the results of each protein target. The assay included internal controls at each stage of the reaction, negative controls to determine background values, and inter-plate controls to account for variability between plates.

ΔCt values were log normalised to what is called 'normalised protein expression' values or NPX. Perseus software (version 1.6.0.7) was used to analyse the vast range of NPX values across patient groups. A Z-score was performed across the entire data set in order to standardise the NPX values.

In order to filter data based on significance of differentially expressed proteins across all patient groups, an analysis of variance (ANOVA) test was carried out with permutation-based false discovery rate (FDR). These proteins were expressed in a heat map with unsupervised clustering.

### Example 1

### Proteins that are significantly differentially expressed between each patient group when classified by response, as calculated by ANOVA

NPX values from all four OLINK panels across each patient group were analysed using Perseus software.

**Table. 2. Statistically significant differences in detected levels of plasma proteins were calculated between each patient group using an ANOVA test with permutation based FDR, where FDR = 0.05 (with the number of randomizations set at 250).**

| **Protein** | **Difference (Non-responder -Responder)** | **- log ANOVA p value (all patient groups)** | **ANOVA p value** | **FDR q value** |
|---|---|---|---|---|
| **CASP-3** | 1.654 | 12.924 | <0.0001 | <0.001 |
| **SELP** | 1.571 | 11.523 | <0.0001 | <0.001 |
| **JAM-A** | 1.589 | 11.496 | <0.0001 | <0.001 |
| **PDGF subunit A** | 1.591 | 10.616 | <0.0001 | <0.001 |
| **ITGB1BP2** | 1.391 | 8.522 | <0.0001 | <0.001 |
| **Dkk-1** | 1.358 | 8.010 | <0.0001 | <0.001 |
| **PECAM-1** | 1.363 | 7.441 | <0.0001 | <0.001 |
| **ANG-1** | 1.326 | 7.229 | <0.0001 | <0.001 |
| **DECR1** | 1.267 | 6.782 | <0.0001 | <0.001 |
| **SOD2** | 1.269 | 6.529 | <0.0001 | <0.001 |
| **DAPP1** | 1.353 | 6.492 | <0.0001 | <0.001 |
| **DCTN1** | 1.288 | 6.278 | <0.0001 | <0.001 |
| **PAI** | 1.300 | 6.022 | <0.0001 | <0.001 |
| **CD40-L** | 1.220 | 5.899 | <0.0001 | <0.001 |
| **HB-EGF** | 1.169 | 5.446 | <0.0001 | 0.001 |
| **HCLS1** | 1.273 | 5.285 | <0.0001 | 0.002 |
| **IRAK4** | 1.173 | 5.008 | <0.0001 | 0.002 |
| **STK4** | 1.214 | 4.892 | <0.0001 | 0.002 |
| **SH2B3** | 1.196 | 4.743 | <0.0001 | 0.002 |
| **NEMO** | 1.034 | 4.686 | <0.0001 | 0.002 |
| **CD84** | 1.065 | 4.605 | <0.0001 | 0.002 |
| **EIF4G1** | 1.175 | 4.584 | <0.0001 | 0.002 |
| **PRDX1** | -0.945 | 4.435 | <0.0001 | 0.002 |
| **TANK** | 1.073 | 4.254 | 0.0001 | 0.004 |
| **CXCL1** | 1.036 | 4.251 | 0.0001 | 0.004 |
| **SIRT2** | 1.122 | 4.168 | 0.0001 | 0.005 |
| **ZBTB16** | 1.028 | 3.645 | 0.0002 | 0.010 |
| **NF2** | 1.112 | 3.639 | 0.0002 | 0.009 |
| **PDGF subunit B** | 1.042 | 3.536 | 0.0003 | 0.010 |
| **SPRY2** | 1.098 | 3.498 | 0.0003 | 0.011 |
| **HEXIM1** | 1.063 | 3.444 | 0.0004 | 0.011 |
| **PPP1R9B** | 1.048 | 3.424 | 0.0004 | 0.012 |
| **PRDX3** | 1.081 | 3.382 | 0.0004 | 0.013 |
| **PAR-1** | 0.866 | 3.241 | 0.0006 | 0.016 |
| **ICA1** | 0.985 | 3.217 | 0.0006 | 0.017 |
| **CCL17** | 0.837 | 3.183 | 0.0007 | 0.017 |
| **STAMPB** | 0.951 | 3.023 | 0.0009 | 0.021 |
| **MGMT** | 0.943 | 2.835 | 0.0015 | 0.026 |
| **SRPK2** | 0.972 | 2.757 | 0.0017 | 0.028 |
| **BLM hydrolase** | 0.945 | 2.678 | 0.0021 | 0.033 |
| **PLXNA4** | 0.951 | 2.630 | 0.0023 | 0.035 |
| **SORT1** | 0.790 | 2.612 | 0.0024 | 0.035 |

### Example 2

### Unsupervised clustering of proteins calculated to be significantly different between DMARD naive (normal) (N), DMARD responder (DR) and DMARD non-responder (DNR) RA patients

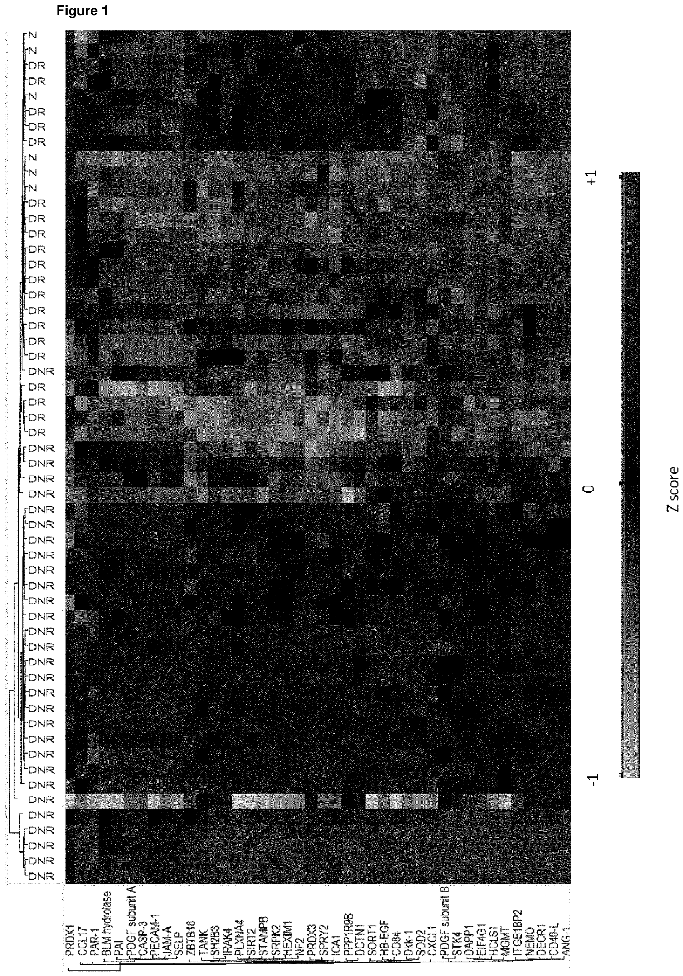

Figure 1 illustrates a heat map showing only the proteins that were significantly different between groups, having an FDR q value of <0.05. Of the 42 proteins calculated as significantly different between patient groups, the majority were upregulated in cDMARD non-responders, compared to responders and cDMARD naive (normal) patients. The unsupervised clustering of the heat map shows a split between a) cDMARD non-responders b) cDMARD naive (normal) and c) cDMARD responders, where just one cDMARD non-responder is clustered among the responders. Furthermore, one cDMARD non-responder shows a reduced protein expression for the majority of the 42 proteins graphed, compared to all other patient samples. The reason for this outlier is unclear from available clinical data.

### Example 3

### Proteins that are significantly differentially expressed between each patient group when classified by disease activity score (DAS), as calculated by ANOVA.

DAS28-ESR scores from clinical data recorded on the same day as sampling were classified according to EULAR criteria (NICE 2009). A DAS28-ESR score of ≥5.1 was classified as 'high' disease activity, >3.2 and ≤5.1 was 'moderate', >2.6 and ≤3.2 was 'low', and <2.6 was classified as 'remission'.

**Table. 3. Each group was analysed by ANOVA with permutation based FDR.**

| **Protein** | **Difference (High disease activity - Remission)** | **- log ANOVA p value (all patient groups)** | **ANOVA p value** | **FDR q value** |
|---|---|---|---|---|
| **ITGB1BP2** | 2.015 | 5.514 | <0.0001 | 0.200 |
| **CD84** | 2.005 | 5.375 | <0.0001 | 0.110 |
| **Dkk-1** | 1.990 | 5.355 | <0.0001 | 0.073 |
| **SOD2** | 2.039 | 5.200 | <0.0001 | 0.057 |
| **SORT1** | 1.961 | 5.158 | <0.0001 | 0.046 |
| **DECR1** | 1.959 | 4.988 | <0.0001 | 0.051 |
| **CASP-3** | 1.813 | 4.985 | <0.0001 | 0.044 |
| **CD40-L** | 1.882 | 4.724 | <0.0001 | 0.044 |
| **PDGF subunit A** | 1.737 | 4.616 | <0.0001 | 0.043 |
| **SELP** | 1.611 | 4.570 | <0.0001 | 0.040 |

### Example 4

### Unsupervised clustering of proteins calculated to be significantly different between RA patients with high, moderate or low disease activity, or who are in remission

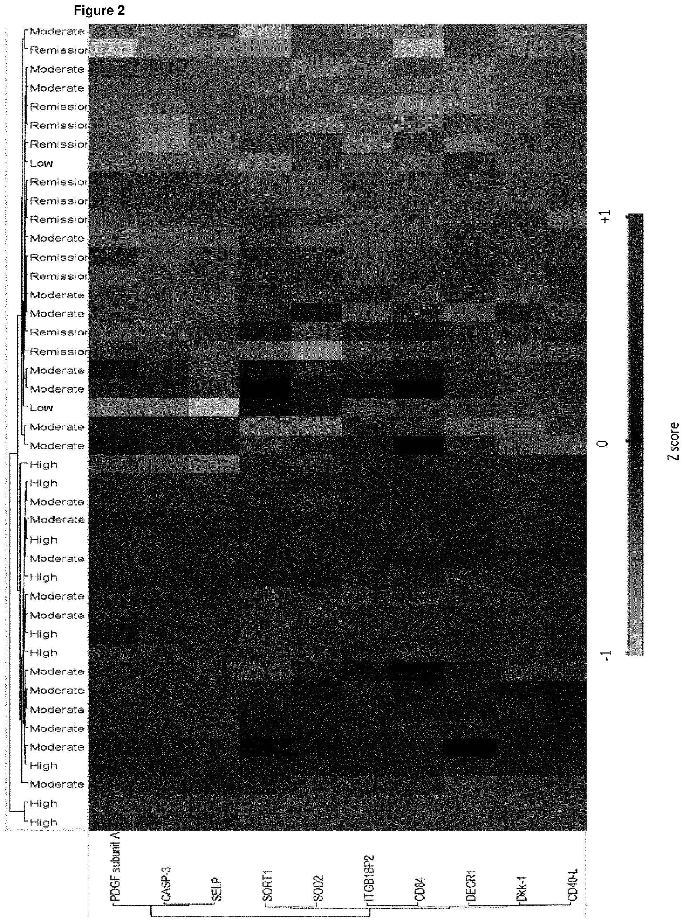

Figure 2 illustrates a heat map with unsupervised clustering showing only the proteins that were significantly different between groups. Of the four protein panels analysed, only ten were statistically significantly different in normalised levels between patient groups.

### Example 5

### Analysis of the top 10 significantly different proteins

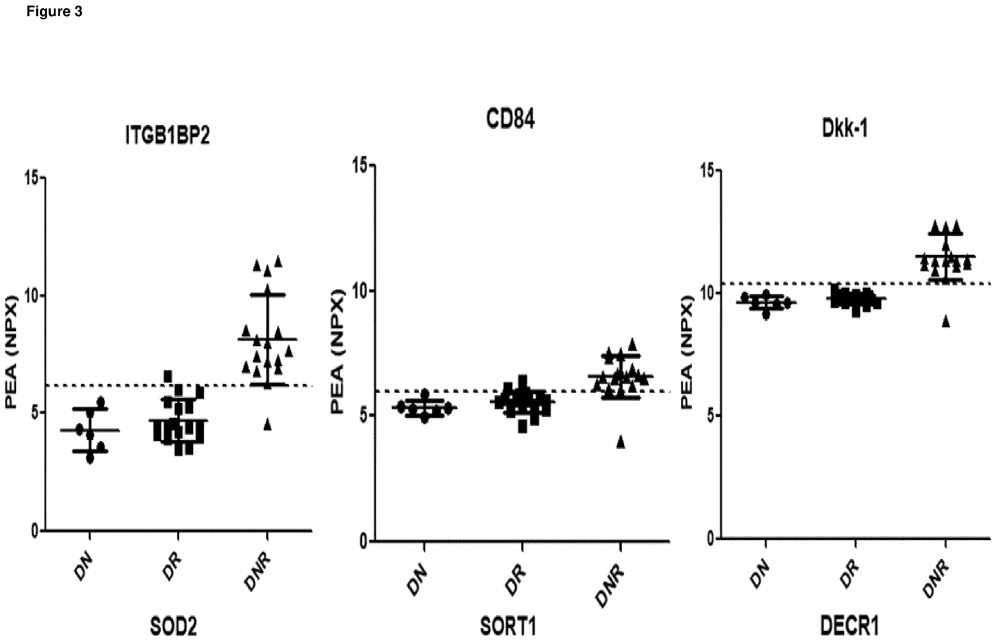

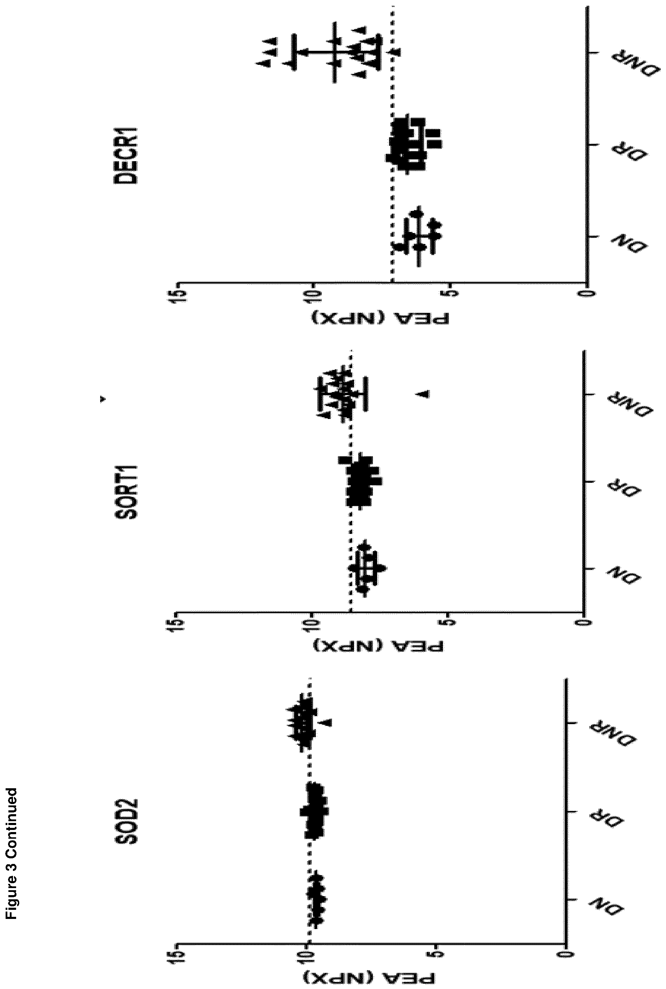

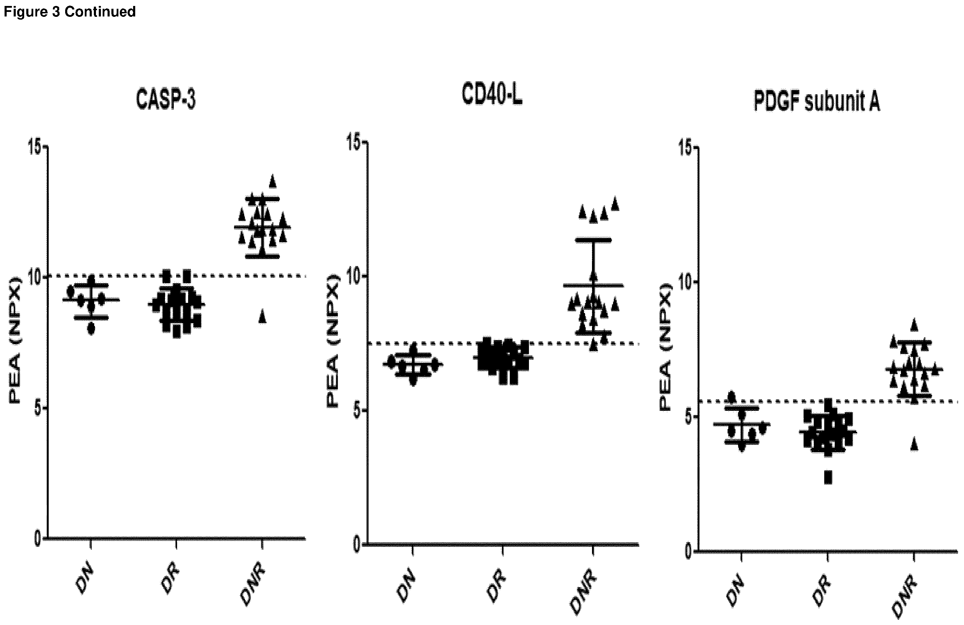

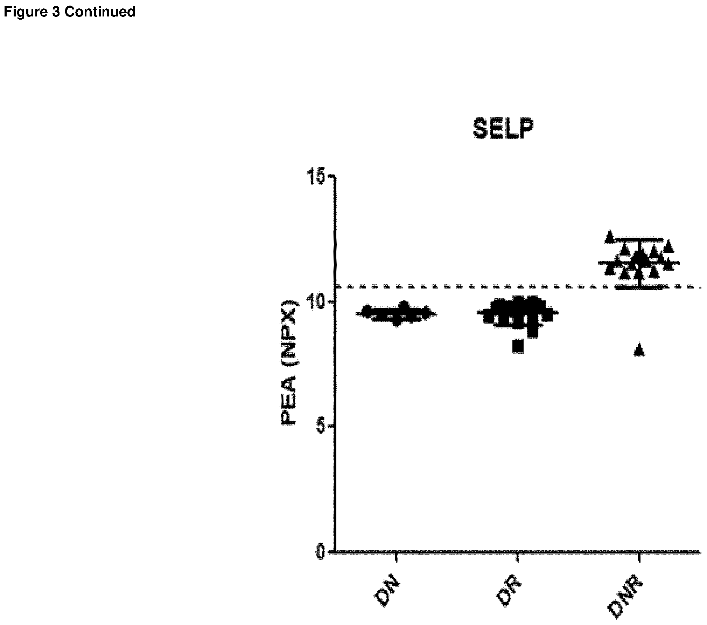

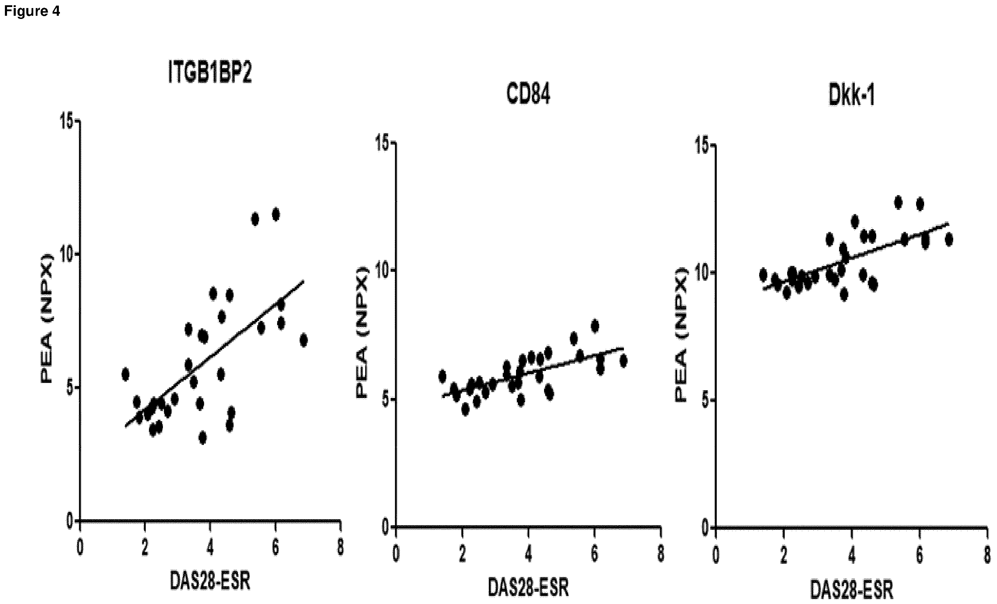

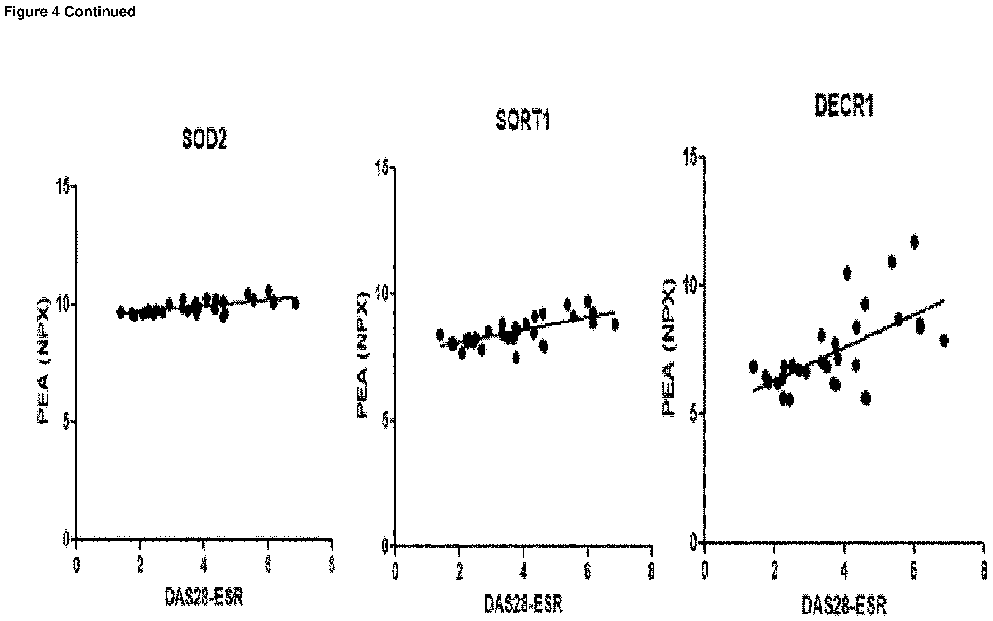

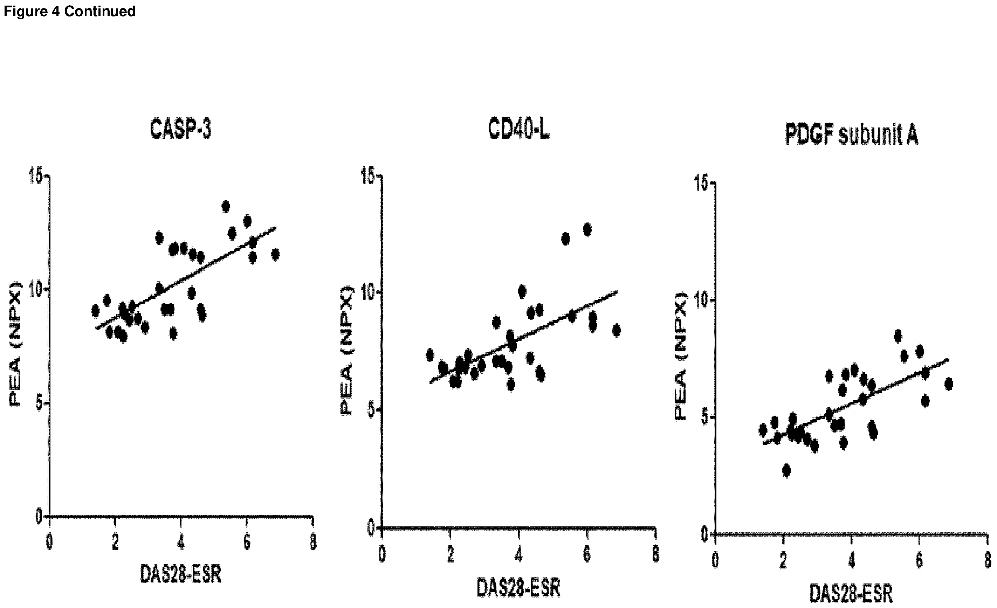

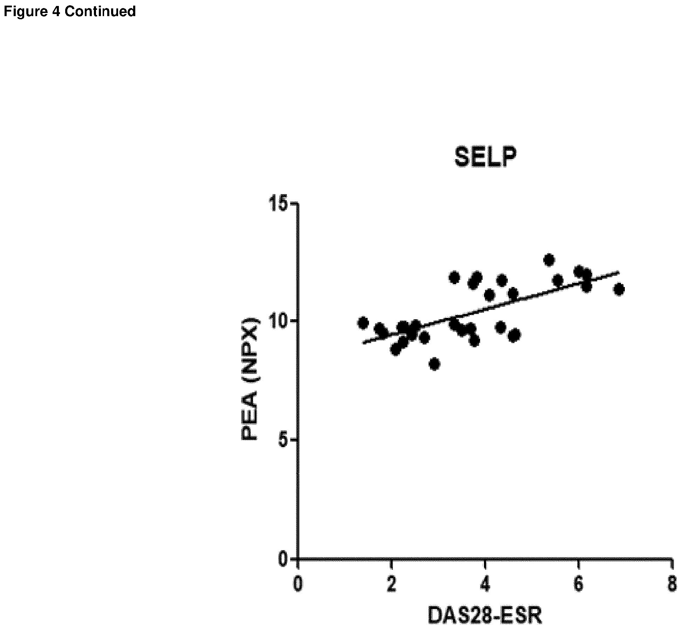

Each of the ten proteins were also significantly different between patient groups when classified by response. Statistical data of the ten proteins is summarised in Table 4 (see also Figure 3 and Figure 4). Proteins were ranked from left to right with the best performing protein on the left, based on AUC score. The benchmark false discovery AUC for this analysis was 0.6251.

**Table. 4. Statistical analysis of top ten proteins that show significant difference across RA patients when grouped by response or DA S28-ESR.**

| **Individual Protein Performance** | **DECR1** | **CD40-L** | **ITGB1B P2** | **CASP-3** | **PDGF subunit A** | **Dkk-1** | **SELP** | **SOD2** | **SORT 1** | **CD84** |
|---|---|---|---|---|---|---|---|---|---|---|
| **p-value** | <0.0001 | <0.0001 | <0.0001 | <0.0001 | <0.0001 | <0.0001 | <0.0001 | <0.0001 | 0.0062 | 0.0001 |
| **AUC** | 1.0000 | 1.0000 | 0.9758 | 0.9550 | 0.9481 | 0.9412 | 0.9412 | 0.9308 | 0.9308 | 0.9204 |
| **Sensitivity** | 1.0000 | 1.0000 | 0.9412 | 1.0000 | 1.0000 | 1.0000 | 1.0000 | 0.9412 | 0.9412 | 0.8824 |
| **Specificity** | 1.0000 | 1.0000 | 0.9412 | 0.9412 | 0.9412 | 0.9412 | 0.9412 | 0.8824 | 0.8824 | 0.8824 |
| **Threshold** | 7.1320 | 7.4976 | 6.2200 | 10.4740 | 5.5889 | 10.4467 | 10.5279 | 9.9025 | 8.5585 | 6.0718 |
| **Correlation with DAS28-ESR (p-value)** | 0.0004 | 0.0002 | < 0.0001 | < 0.0001 | < 0.0001 | < 0.0001 | < 0.0001 | < 0.0001 | < 0.0001 | < 0.0001 |
| **Correlation with DAS28-ESR (r-value)** | 0.6010 | 0.6273 | 0.6577 | 0.7118 | 0.6971 | 0.6853 | 0.6700 | 0.6515 | 0.6566 | 0.6748 |

Proteins are ranked from left to right, with the best performing protein on the left, based on AUC score. ROC curve analysis of each protein when a cut-off (y-intercept) value is applied to distinguish RA cDMARD responders and non-responders (Figure 3). Also shown is correlation data of each NPX value with DAS28-ESR score (Figure 4). We considered the effect of using combinations of proteins for classification. Proteins acting in combination all achieved an AUC score of 1. In order to establish whether this was due to overfitting, we generated nonsense data by randomly swapping patients between the responder and non-responder groups sufficiently to obtain decorrelated nonsense data. We then optimised classifiers to the nonsense data using the same protein combinations and calculated the corresponding AUC score. This was repeated twenty times and the mean AUC was calculated. The results can be seen in Table 5 where the classification AUC scores obtained using true data significantly outperforms the overfitting rate which is equal to the mean classification AUCs obtained using the nonsense data.

**Table. 5. Statistical analysis of the collective protein performance of the top ten proteins that show significant difference across RA patients when grouped by response or DAS28-ESR.**

| **Collective Protein Performance** | **DECR1** | **CD40-L** | **ITGB1BP 2** | **CASP-3** | **PDGF subunit A** | **Dkk-1** | **SELP** | **SOD2** | **SORT1** | **CD84** |
|---|---|---|---|---|---|---|---|---|---|---|
| **AUC** | 1 | 1 | 1 | 1 | 1 | 1 | 1 | 1 | 1 | 1 |
| **Sensitivity** | 1 | 1 | 1 | 1 | 1 | 1 | 1 | 1 | 1 | 1 |
| **Specificity** | 1 | 1 | 1 | 1 | 1 | 1 | 1 | 1 | 1 | 1 |
| **Benchmark AUC** | 0.625 | | | | | | | | | |
| **Benchmark SE** | 0.013 | | | | | | | | | |
| **Benchmark Sensitivity** | 0.541 | 0.5411 | 0.5411 | 0.541 | 0.541 | 0.541 | 0.54 | 0.541 | 0.541 | 0.541 |
| **Benchmark Specificity** | 0.617 | 0.6176 | 0.6176 | 0.617 | 0.6176 | 0.6176 | 0.617 | 0.6176 | 0.6176 | 0.617 |
| **Threshold** | -28.84 | -3.120 | -2.540 | -1.31 | -1.2572 | -1.275 | -1.28 | -1.2760 | -0.680 | -0.605 |
| | 205.70 | -4.427 | -2.3013 | -0.55 | -0.439 | -0.472 | -0.46 | -0.4470 | -0.315 | -0.325 |
| | 0 | 55.773 | -2.0427 | 0.1429 | 0.3053 | 0.2642 | 0.283 | 0.3107 | 0.0233 | -0.0574 |
| | 0 | 0 | 49.256 | -0.913 | -0.560 | -0.494 | -0.332 | -0.3449 | -0.3096 | -0.3124 |
| | 0 | 0 | 0 | 22.535 | -0.518 | -0.495 | -0.378 | -0.3728 | -0.3396 | -0.3677 |
| | 0 | 0 | 0 | 0 | 19.477 | -0.125 | -0.024 | -0.0450 | -0.0575 | -0.0381 |
| | 0 | 0 | 0 | 0 | 0 | 20.538 | -0.292 | -0.301 | -0.309 | -0.322 |
| | 0 | 0 | 0 | 0 | 0 | 0 | 20.122 | 0.0805 | 0.0724 | 0.1029 |
| | 0 | 0 | 0 | 0 | 0 | 0 | 0 | 19.349 | 0.6848 | 0.5951 |
| | 0 | 0 | 0 | 0 | 0 | 0 | 0 | 0 | 9.8592 | 0.2706 |
| | 0 | 0 | 0 | 0 | 0 | 0 | 0 | 0 | 0 | 8.8411 |

Each column presents the results for the classifier obtained for the protein labelled in the column in combination with any proteins to the left of the column. As we move to the right of the table, incrementally more proteins are included in the classifier. AUC, sensitivity and specificity are shown for the protein combinations applied to true data. Also shown are the overfitting rate (mean benchmark AUC) and the standard error in the benchmark AUC, obtained from twenty iterations of calculating nonsense data, the mean sensitivity and specificity of the classifiers obtained for nonsense data and the thresholds identified which grow in dimension with increasing numbers of proteins.

One of the key aims of this work was to discover circulating protein biomarkers that may aid in differentiating clinical response to cDMARD treatment in RA. Of the 4 protein panels tested from OLINK (total number = 384), 42 proteins were significantly differentially expressed between cDMARD naive (normal), cDMARD responder and cDMARD non-responder RA patients. When classifying patients based on disease activity as determined by EULAR criteria, 10 proteins were shown to be differentially expressed between patient groups. Interestingly, these 10 proteins were also included in the 42 proteins previously shown to be differentially expressed when patients were classified by response. This suggests that these 10 proteins have the potential of distinguishing both disease activity and response to cDMARD treatment.

When a threshold is applied, each of the individual 10 proteins demonstrates good sensitivity (>88%) and specificity (>88%) in distinguishing responders from non-responders.

Overall, considering the results of the individual protein analysis and the combined protein analysis, DECR1 and CD40-L are the most optimal individual proteins in distinguishing response. No incremental benefit was observed when combining these proteins with each other or the others on the list (Table 5).

## Claims

1. An *in vitro* method of predicting response to treatment with a disease-modifying anti-rheumatic drug (DMARD), and/or classifying disease activity in a subject with rheumatoid arthritis, the method comprising the steps of:
(a) determining the quantitative level of two or more biomarkers in a biological sample from the subject; and
(b) predicting response to treatment with a DMARD, and/or classifying disease activity based on the quantitative level of the biomarkers in the biological sample;
wherein the biomarkers are selected from DECR1 and CD40-L.

2. The method according to Claim 1, wherein the biomarkers for predicting response to treatment with a DMARD, and/or classifying disease activity is a gene having a GenBank Accession Version Number selected from of AK300069.1 and L07414.1.

3. The method according to Claim 1, wherein the or each biomarkers for predicting response to treatment with a DMARD, and/or classifying disease activity is a protein having a UniProt ID selected from of Q16698 and P29965.

4. The method according to Claim 1, wherein the biomarkers further comprise one or more of ITGB1BP2, CASP-3, PDFG subunit A, Dkk-1, SELP, SOD2, SORT1 and CD84.

5. The method according to Claim 2, wherein the biomarker for predicting response to treatment with a DMARD, and/or classifying disease activity is a gene having a GenBank Accession Version Number further comprise one or more of BC108901.2, AJ413269.1 , AH002928.2, AH009834.2, AL022146.1 , X07834.1 , AL390252.9 and AF054816.1.

6. The method according to Claim 3, wherein the biomarker for predicting response to treatment with a DMARD, and/or classifying disease activity is a protein having a UniProt ID further comprise one or more of Q9UKP3, P42574, P04085, 094907, P16109, P04179, Q99523 and Q9UIB8.

7. The method according to Claim 4, wherein the method is a method for predicting response to treatment with a DMARD, and biomarkers further comprise JAM-A, PECAM-1, ANG-1, DAPP1, DCTN1 and PAI.

8. The method according to Claim 7, wherein the biomarker for predicting response to treatment with a DMARD further comprise HB-EGF, HCLS1, IRAK4, STK4, SH2B3, NEMO, EIF4G1, PRDX1, TANK, CXCL1, SIRT2, ZBTB16, NF2, PDGF subunit B, SPRY2, HEXIM1, PPP1R9B, PRDX3, PAR-1, ICA1, CCL17, STAMPB, MGMT, SRPK2, BML hydrolase and PLXNA4.

9. The method according to Claim 8, wherein the biomarker for predicting response to treatment with a DMARD is a gene having a GenBank Accession Version Number further comprise one or more of AK300069.1, AJ413269.1, AL022146.1, AL136649.1, AH002928.2, BC108901.2, BC107046.2, JQ287500.1, AY124380.1, X07834.1, AF178987.1 AK314352.1, X04731.1, AK222896.1, AC004634.1, AK312750.1, AY340965.1, Z93016.2, BC136451.1, AJ271718.1, AF054816.1, AK131407.1, CR407652.1, AC009299.5, BT006880.1, KF032391.1, BC029812.1, Y18000.1, M12783.1, AL354668.13, BC006460.1, KF495721.1, BT020007.1, BC002464.2, U37183.1, CH471092.1, CH471053.2, M60761.1, AC004884.1, AK312896.1, AC009365.9 and AL390252.9.

10. The method according to Claim 8, wherein the biomarker for predicting response to treatment with a DMARD is a protein having a UniProt ID further comprise one or more of Q16698, P42574, P16109, Q9Y624, P04085, Q9UKP3, Q9UBT3, P16284, Q15389, P04179, Q9UN19, Q14203, P05121, P25942, Q99075, P14317, Q9NWZ3, Q13043, Q9UQQ2, Q9Y6K9, Q9UIB8, Q04637, Q06830, Q92844, P09341, Q8IXJ6, Q05516, P35240, P01127, O43597, O94992, Q96SB3, P30048, P25116, Q05084, Q92583, O95630, P16455, P78362, Q13867, Q9HCM2 and Q99523.

11. The method according to Claim 8, wherein the determining step (a) further comprises determining the quantitative level in a first set of respective biomarkers(s), wherein the quantitative level of the first set of biomarkers is greater than the quantitative level of the same respective biomarkers(s) in a normal sample, which is indicative of a non-responder, the first set of biomarkers is selected from any one or more of DECR1, CASP-3, SELP, JAM-A, PDFG subunit A, ITGB1BP2, Dkk-1, PECAM-1, ANG-1, SOD2, DAPP1, DCTN1, PAI, CD40-L, HB-EGF, HCLS1, IRAK4, STK4, SH2B3, NEMO, CD84, EIF4G1, PRDX1, TANK, CXCL1, SIRT2, ZBTB16, NF2, PDGF subunit B, SPRY2, HEXIM1, PPP1R9B, PRDX3, PAR-1, ICA1, CCL17, STAMPB, MGMT, SRPK2, BML hydrolase, PLXNA4 and SORT1; or wherein the determining step (a) further comprises determining the quantitative level in a second set of respective biomarkers(s), wherein the quantitative level of the second set of biomarkers is less than the quantitative level of the same respective biomarkers(s) in a normal sample, which is indicative of a non-responder, the second set of biomarkers is selected from any one or more of DECR1, CD40-L, ITGB1BP2, CASP-3, PDGF subunit A, Dkk-1, SELP, SOD2, SORT1 and CD84.

12. The method according Claim 11, wherein the quantitative level of the or each biomarker is higher than a threshold value and is indicative of a non-responder, wherein the respective threshold value of the or each biomarker is:
| **Biomarker** | **Threshold value** |
|---|---|
| DECR1 | 7.1320 |
| | |
|---|---|
| CASP-3 | 10.4740 |
| SELP | 10.5279 |
| PDGF subunit A | 5.5889 |
| ITGB1BP2 | 6.2200 |
| Dkk-1 | 10.4467 |
| SOD2 | 9.9025 |
| CD40-L | 7.4976 |
| CD84 | 6.0718 |
| SORT1 | 8.5585 |

13. The method according to Claim 11, wherein the quantitative level of the or each biomarker is lower than a threshold value and is indicative of a non-responder, wherein the respective threshold value of the or each biomarker is:
| **Biomarker** | **Threshold value** |
|---|---|
| DECR1 | 7.1320 |
| CASP-3 | 10.4740 |
| SELP | 10.5279 |
| PDGF subunit A | 5.5889 |
| ITGB1BP2 | 6.2200 |
| Dkk-1 | 10.4467 |
| SOD2 | 9.9025 |
| CD40-L | 7.4976 |
| CD84 | 6.0718 |
| SORT1 | 8.5585 |

14. The method according to any one of Claims 1-6, wherein the quantitative level of the or each biomarker is between two threshold values and is indicative of high disease activity, wherein the respective threshold values of the or each biomarker are:
| **Biomarker** | **Threshold value** |
|---|---|
| DECR1 | 7.8984 - 11.6893 |
| CD40-L | 8.4728 - 12.7503 |
| ITGB1BP2 | 6.7802 - 11.4759 |
| CASP-3 | 11.4114 - 13.6768 |
| PDFG subunit A | 5.7334 - 8.4577 |
| Dkk-1 | 11.2034 - 12.7568 |
| SELP | 11.3715 - 12.6136 |
| | |
|---|---|
| SOD2 | 10.0471 - 10.5467 |
| SORT1 | 8.7990 - 9.7006 |
| CD84 | 6.1830 - 7.8683 |
or wherein the quantitative level of the or each biomarker is between two threshold values and is indicative of moderate disease activity, wherein the respective threshold values of the or each biomarker are:
| **Biomarker** | **Threshold values** |
|---|---|
| DECR1 | 5.6510 - 10.5039 |
| CD40-L | 6.1622- 10.1159 |
| ITGB1BP2 | 3.1210 - 8.5301 |
| CASP-3 | 8.0945 - 12.2662 |
| PDFG subunit A | 3.9517 - 7.0510 |
| Dkk-1 | 9.1861 - 12.0037 |
| SELP | 9.2287 - 11.9099 |
| SOD2 | 9.5039 - 10.2607 |
| SORT1 | 7.5225 - 9.2595 |
| CD84 | 4.9694 - 6.8399 |
or wherein the quantitative level of the or each biomarker is between two threshold values and is indicative of low disease activity, wherein the respective threshold values of the or each biomarker are:
| **Biomarker** | **Threshold values** |
|---|---|
| DECR1 | 6.6490 - 6.7309 |
| CD40-L | 6.5871 - 6.9401 |
| ITGB1BP2 | 4.1040 - 4.5746 |
| CASP-3 | 8.3368 - 8.7173 |
| PDFG subunit A | 3.8283 - 4.0907 |
| Dkk-1 | 9.6015 - 9.8410 |
| SELP | 8.2174 - 9.3414 |
| SOD2 | 9.6542 - 9.9702 |
| SORT1 | 7.7980 - 8.5272 |
| CD84 | 5.2689 - 5.5881 |
or wherein the quantitative level of the or each biomarker is between two threshold values and is indicative of in remission, wherein the respective threshold values of the or each biomarker are:
| **Biomarker** | **Threshold value** |
|---|---|
| DECR1 | 5.6064 - 6.9173 |
| CD40-L | 6.2510 - 7.3891 |
| ITGB1BP2 | 3.4508 - 5.5040 |
| CASP-3 | 7.9543 - 9.2353 |
| PDFG subunit A | 2.7648 - 4.9645 |
| Dkk-1 | 9.2598 - 9.9684 |
| SELP | 8.8272 - 9.9551 |
| SOD2 | 9.5195 - 9.7582 |
| SORT1 | 7.6829 - 8.4218 |
| CD84 | 4.6188 - 5.8750 |

15. The method according to any one of Claims 1-14, wherein the biological sample is selected from whole blood, serum, plasma, urine, interstitial fluid, peritoneal fluid, cervical swab, tears, saliva, buccal swab, skin, brain tissue, and cerebrospinal fluid.

## Patentansprüche

1. In-vitro-Verfahren zum Vorhersagen von Reaktion auf Behandlung mit einem krankheitsmodifizierenden Antirheumatikum (disease-modifying anti-rheumatic drug, DMARD) und/oder Klassifizieren der Krankheitsaktivität bei einem Subjekt mit rheumatoider Arthritis, wobei das Verfahren die folgenden Schritte umfasst:
(a) Bestimmen des quantitativen Niveaus von zwei oder mehr Biomarkern in einer biologischen Probe des Subjekts; und
(b) Vorhersagen von Reaktion auf Behandlung mit einem DMARD, und/oder Klassifizieren der Krankheitsaktivität basierend auf dem quantitativen Niveau der Biomarker in der biologischen Probe;
wobei die Biomarker aus DECR1 und CD40-L ausgewählt sind.

2. Verfahren nach Anspruch 1, wobei es sich bei den Biomarkern zum Vorhersagen von Reaktion auf Behandlung mit einem DMARD und/oder zum Klassifizieren der Krankheitsaktivität um ein Gen handelt, das eine GenBank-Zugangsversionsnummer aufweist, die aus AK300069.1 und L07414.1 ausgewählt ist.

3. Verfahren nach Anspruch 1, wobei es sich bei dem oder jedem Biomarker zum Vorhersagen von Reaktion auf Behandlung mit einem DMARD und/oder zum Klassifizieren der Krankheitsaktivität um ein Protein handelt, das eine UniProt-ID aufweist, die aus Q16698 und P29965 ausgewählt ist.

4. Verfahren nach Anspruch 1, wobei die Biomarker ferner eines oder mehrere von ITGB1BP2, CASP-3, PDFG-Untereinheit A, Dkk-1, SELP, SOD2, SORT1 und CD84 umfassen.

5. Verfahren nach Anspruch 2, wobei es sich bei dem Biomarker zum Vorhersagen von Reaktion auf Behandlung mit einem DMARD und/oder zum Klassifizieren der Krankheitsaktivität um ein Gen handelt, das eine GenBank-Zugangsversionsnummer aufweist, die ferner eines oder mehrere von BC108901.2, AJ413269.1, AH002928.2, AH009834.2, AL022146.1, X07834.1, AL390252.9 und AF054816.1 umfasst.

6. Verfahren nach Anspruch 3, wobei es sich bei dem Biomarker zum Vorhersagen von Reaktion auf Behandlung mit einem DMARD und/oder zum Klassifizieren der Krankheitsaktivität um ein Protein handelt, das eine UniProt-ID aufweist, die ferner eines oder mehrere von Q9UKP3, P42574, P04085, 094907, P16109, P04179, Q99523 und Q9UIB8 umfasst.

7. Verfahren nach Anspruch 4, wobei das Verfahren ein Verfahren zum Vorhersagen von Reaktion auf Behandlung mit einem DMARD ist und die Biomarker ferner JAM-A, PECAM-1, ANG-1, DAPP1, DCTN1 und PAI umfassen.

8. Verfahren nach Anspruch 7, wobei der Biomarker zum Vorhersagen von Reaktion auf Behandlung mit einem DMARD ferner HB-EGF, HCLS1, IRAK4, STK4, SH2B3, NEMO, EIF4G1, PRDX1, TANK, CXCL1, SIRT2, ZBTB16, NF2, PDGF-Untereinheit B, SPRY2, HEXIM1, PPP1R9B, PRDX3, PAR-1, ICA1, CCL17, STAMPB, MGMT, SRPK2, BML-Hydrolase und PLXNA4 umfasst.

9. Verfahren nach Anspruch 8, wobei der Biomarker zum Vorhersagen von Reaktion auf Behandlung mit einem DMARD ein Gen ist, das eine GenBank-Zugangsversionsnummer aufweist, die ferner eines oder mehrere von AK300069.1, AJ413269.1, AL022146.1, AL136649.1, AH002928.2, BC108901.2, BC107046.2, JQ287500.1, AY124380.1, X07834.1, AF178987.1 AK314352.1, X04731.1, AK222896.1, AC004634.1, AK312750.1, AY340965.1, Z93016.2, BC136451.1, AJ271718.1, AF054816.1, AK131407.1, CR407652.1, AC009299.5, BT006880.1, KF032391.1, BC029812.1, Y18000.1, M12783.1, AL354668.13, BC006460.1, KF495721.1, BT020007.1, BC002464.2, U37183.1, CH471092.1, CH471053.2, M60761.1, AC004884.1, AK312896.1, AC009365.9 und AL390252.9 umfasst.

10. Verfahren nach Anspruch 8, wobei der Biomarker zum Vorhersagen von Reaktion auf Behandlung mit einem DMARD ein Protein ist, das eine UniProt-ID aufweist, die ferner eines oder mehrere von Q16698, P42574, P16109, Q9Y624, P04085, Q9UKP3, Q9UBT3, P16284, Q15389, P04179, Q9UN19, Q14203, P05121, P25942, Q99075, P14317, Q9NWZ3, Q13043, Q9UQQ2, Q9Y6K9, Q9UIB8, Q04637, Q06830, Q92844, P09341, Q8IXJ6, Q05516, P35240, P01127, O43597, O94992, Q96SB3, P30048, P25116, Q05084, Q92583, O95630, P16455, P78362, Q13867, Q9HCM2 und Q99523 umfasst.

11. Verfahren nach Anspruch 8, wobei der Bestimmungsschritt (a) ferner das Bestimmen des quantitativen Niveaus in einem ersten Satz von jeweiligen Biomarker(n) bestimmt, wobei das quantitative Niveau des ersten Satzes von Biomarkern größer ist als das quantitative Niveau desselben/derselben jeweiligen Biomarker in einer normalen Probe, was auf einen Nonresponder hinweist, wobei der erste Satz von Biomarkern aus einem oder mehreren von DECR1, CASP-3, SELP, JAM-A, PDFG-Untereinheit A, ITGB1BP2, Dkk-1, PECAM-1, ANG-1, SOD2, DAPP1, DCTN1, PAI, CD40-L, HB-EGF, HCLS1, IRAK4, STK4, SH2B3, NEMO, CD84, EIF4G1, PRDX1, TANK, CXCL1, SIRT2, ZBTB16, NF2, PDGF-Untereinheit B, SPRY2, HEXIM1, PPP1R9B, PRDX3, PAR-1, ICA1, CCL17, STAMPB, MGMT, SRPK2, BML-Hydrolase, PLXNA4 und SORT1 ausgewählt ist; oder wobei der Bestimmungsschritt (a) ferner das Bestimmen des quantitativen Niveaus in einem zweiten Satz von jeweiligen Biomarker(n) umfasst, wobei das quantitative Niveau des zweiten Satzes von Biomarkern geringer ist als das quantitative Niveau desselben/derselben jeweiligen Biomarker in einer normalen Probe, was auf einen Nonresponder hinweist, wobei der zweite Satz von Biomarkern aus einem oder mehreren von DECR1, CD40-L, ITGB1BP2, CASP-3, PDGF-Untereinheit A, Dkk-1, SELP, SOD2, SORT1 und CD84 ausgewählt ist.

12. Verfahren nach Anspruch 11, wobei das quantitative Niveau des oder jedes Biomarkers höher ist als ein Schwellenwert und auf einen Nonresponder hinweist, wobei der jeweilige Schwellenwert des oder jedes Biomarkers wie folgt lautet:
| **Biomarker** | **Schwellenwert** |
|---|---|
| DECR1 | 7,1320 |
| CASP-3 | 10, 4740 |
| SELP | 10,5279 |
| PDGF-Untereinhei t A | 5,5889 |
| ITGB1BP2 | 6,2200 |
| Dkk-1 | 10,4467 |
| SOD2 | 9,9025 |
| CD40-L | 7,4976 |
| CD84 | 6, 0718 |
| SORT1 | 8,5585 |

13. Verfahren nach Anspruch 11, wobei das quantitative Niveau des oder jedes Biomarkers geringer ist als ein Schwellenwert und auf einen Nonresponder hinweist, wobei der jeweilige Schwellenwert des oder jedes Biomarkers wie folgt lautet:
| **Biomarker** | **Schwellenwert** |
|---|---|
| DECR1 | 7,1320 |
| CASP-3 | 10, 4740 |
| SELP | 10,5279 |
| PDGF-Untereinhei t A | 5,5889 |
| ITGB1BP2 | 6,2200 |
| Dkk-1 | 10,4467 |
| SOD2 | 9,9025 |
| CD40-L | 7,4976 |
| CD84 | 6,0718 |
| SORT1 | 8,5585 |

14. Verfahren nach einem der Ansprüche 1-6, wobei das quantitative Niveau des oder jedes Biomarkers zwischen zwei Schwellenwerten liegt und auf eine hohe Krankheitsaktivität hinweist, und wobei die jeweiligen Schwellenwerte des oder jedes Biomarkers wie folgt lauten:
| **Biomarker** | **Schwellenwert** |
|---|---|
| DECR1 | 7,8984 - 11,6893 |
| CD40-L | 8,4728 - 12,7503 |
| ITGB1BP2 | 6,7802 - 11,4759 |
| CASP-3 | 11,4114 - 13,6768 |
| PDFG-Untereinhei t A | 5,7334 - 8,4577 |
| Dkk-1 | 11,2034 - 12,7568 |
| SELP | 11,3715 - 12,6136 |
| SOD2 | 10,0471 - 10,5467 |
| SORT1 | 8,7990 - 9,7006 |
| CD84 | 6,1830 - 7,8683 |
oder wobei das quantitative Niveau des oder jedes Biomarkers zwischen zwei Schwellenwerten liegt und auf eine moderate Krankheitsaktivität hinweist, und wobei die jeweiligen Schwellenwerte des oder jedes Biomarkers wie folgt lauten:
| **Biomarker** | **Schwellenwerte** |
|---|---|
| DECR1 | 5,6510 - 10,5039 |
| CD40-L | 6,1622 - 10,1159 |
| ITGB1BP2 | 3,1210 - 8,5301 |
| CASP-3 | 8,0945 - 12,2662 |
| PDFG-Untereinhei t A | 3,9517 - 7,0510 |
| Dkk-1 | 9,1861 - 12,0037 |
| SELP | 9,2287 - 11,9099 |
| SOD2 | 9,5039 - 10,2607 |
| SORT1 | 7,5225 - 9,2595 |
| CD84 | 4,9694 - 6,8399 |
oder wobei das quantitative Niveau des oder jedes Biomarkers zwischen zwei Schwellenwerten liegt und auf eine geringe Krankheitsaktivität hinweist, und wobei die jeweiligen Schwellenwerte des oder jedes Biomarkers wie folgt lauten:
| **Biomarker** | **Schwellenwerte** |
|---|---|
| DECR1 | 6,6490 - 6,7309 |
| CD40-L | 6,5871 - 6,9401 |
| ITGB1BP2 | 4,1040 - 4,5746 |
| CASP-3 | 8,3368 - 8,7173 |
| PDFG- | 3,8283 - 4,0907 |
| Untereinhei t A | |
|---|---|
| Dkk-1 | 9,6015 - 9,8410 |
| SELP | 8,2174 - 9,3414 |
| SOD2 | 9,6542 - 9,9702 |
| SORT1 | 7,7980 - 8,5272 |
| CD84 | 5,2689 - 5,5881 |
oder wobei das quantitative Niveau des oder jedes Biomarkers zwischen zwei Schwellenwerten liegt und auf Remission hinweist, und wobei die jeweiligen Schwellenwerte des oder jedes Biomarkers wie folgt lauten:
| **Biomarker** | **Schwellenwert** |
|---|---|
| DECR1 | 5,6064 - 6,9173 |
| CD40-L | 6,2510 - 7,3891 |
| ITGB1BP2 | 3,4508 - 5,5040 |
| CASP-3 | 7,9543 - 9,2353 |
| PDFG-Untereinhei t A | 2,7648 - 4,9645 |
| Dkk-1 | 9,2598 - 9,9684 |
| SELP | 8,8272 - 9,9551 |
| SOD2 | 9,5195 - 9,7582 |
| SORT1 | 7,6829 - 8,4218 |
| CD84 | 4,6188 - 5,8750 |

15. Verfahren nach einem der Ansprüche 1-14, wobei die biologische Probe ausgewählt ist aus Vollblut, Serum, Plasma, Urin, interstitieller Flüssigkeit, Peritonealflüssigkeit, Gebärmutterhalsabstrich, Tränen, Speichel, Wangenabstrich, Haut, Hirngewebe und Zerebrospinalflüssigkeit.

## Revendications

1. Procédé *in vitro* de prédiction de la réponse au traitement par un médicament antirhumatismal modificateur de la maladie (DMARD), et/ou de classification de l'activité de la maladie chez un sujet atteint de polyarthrite rhumatoïde, le procédé comprenant les étapes de :
(a) détermination du niveau quantitatif de deux biomarqueurs ou plus dans un échantillon biologique du sujet ; et
(b) prédiction de la réponse au traitement par un DMARD, et/ou classification de l'activité de la maladie sur la base du niveau quantitatif des biomarqueurs dans l'échantillon biologique ;
dans lequel les biomarqueurs sont choisis parmi DECR1 et CD40-L.

2. Procédé selon la revendication 1, dans lequel les biomarqueurs permettant de prédire la réponse au traitement avec un DMARD et/ou de classer l'activité de la maladie sont un gène ayant un numéro de version d'accès GenBank choisi parmi AK300069.1 et L07414.1.

3. Procédé selon la revendication 1, dans lequel le ou chaque biomarqueur permettant de prédire la réponse au traitement avec un DMARD et/ou de classer l'activité de la maladie est une protéine ayant un identifiant UniProt choisi parmi Q16698 et P29965.

4. Procédé selon la revendication 1, dans lequel les biomarqueurs comprennent également un ou plusieurs parmi ITGB1BP2, CASP-3, la sous-unité A de PDFG, Dkk-1, SELP, SOD2, SORT1 et CD84.

5. Procédé selon la revendication 2, dans lequel le biomarqueur permettant de prédire la réponse au traitement par un DMARD et/ou de classer l'activité de la maladie est un gène ayant un numéro de version d'accès GenBank comprenant également un ou plusieurs parmi BC108901.2, AJ413269.1, AH002928.2, AH009834.2, AL022146.1, X07834.1, AL390252.9 et AF054816.1.

6. Procédé selon la revendication 3, dans lequel le biomarqueur permettant de prédire la réponse au traitement par un DMARD et/ou de classer l'activité de la maladie est une protéine ayant un identifiant UniProt comprenant également un ou plusieurs parmi Q9UKP3, P42574, P04085, 094907, P16109, P04179, Q99523 et Q9UIB8.

7. Procédé selon la revendication 4, dans lequel le procédé est un procédé de prédiction de la réponse au traitement par un DMARD, et les biomarqueurs comprennent également JAM-A, PECAM-1, ANG-1, DAPP1, DCTN1 et PAI.

8. Procédé selon la revendication 7, dans lequel le biomarqueur permettant de prédire la réponse au traitement par un DMARD comprend également HB-EGF, HCLS1, IRAK4, STK4, SH2B3, NEMO, EIF4G1, PRDX1, TANK, CXCL1, SIRT2, ZBTB16, NF2, la sous-unité B de PDGF, SPRY2, HEXIM1, PPP1R9B, PRDX3, PAR-1, ICA1, CCL17, STAMPB, MGMT, SRPK2, l'hydrolase BML et PLXNA4.

9. Procédé selon la revendication 8, dans lequel le biomarqueur permettant de prédire la réponse au traitement par un DMARD est un gène ayant un numéro de version d'accès GenBank comprenant également un ou plusieurs parmi AK300069.1, AJ413269.1, AL022146.1, AL136649.1, AH002928.2, BC108901.2, BC107046.2, JQ287500.1, AY124380.1, X07834.1, AF178987.1 AK314352.1, X04731.1, AK222896.1, AC004634.1, AK312750.1, AY340965.1, Z93016.2, BC136451.1, AJ271718.1, AF054816.1, AK131407.1, CR407652.1, AC009299.5, BT006880.1, KF032391.1, BC029812.1, Y18000.1, M12783.1, AL354668.13, BC006460.1, KF495721.1, BT020007.1, BC002464.2, U37183.1, CH471092.1, CH471053.2, M60761.1, AC004884.1, AK312896.1, AC009365.9 et AL390252.9.

10. Procédé selon la revendication 8, dans lequel le biomarqueur permettant de prédire la réponse au traitement par un DMARD est une protéine ayant un identifiant UniProt comprenant également un ou plusieurs parmi Q16698, P42574, P16109, Q9Y624, P04085, Q9UKP3, Q9UBT3, P16284, Q15389, P04179, Q9UN19, Q14203, P05121, P25942, Q99075, P14317, Q9NWZ3, Q13043, Q9UQQ2, Q9Y6K9, Q9UIB8, Q04637, Q06830, Q92844, P09341, Q8IXJ6, Q05516, P35240, P01127, O43597, O94992, Q96SB3, P30048, P25116, Q05084, Q92583, O95630, P16455, P78362, Q13867, Q9HCM2 et Q99523.

11. Procédé selon la revendication 8, dans lequel l'étape de détermination (a) comprend également la détermination du niveau quantitatif dans un premier ensemble de biomarqueurs respectifs, dans lequel le niveau quantitatif du premier ensemble de biomarqueurs est supérieur au niveau quantitatif du ou des mêmes biomarqueurs respectifs dans un échantillon normal, ce qui indique l'absence de réponse, le premier ensemble de biomarqueurs est choisi parmi un ou plusieurs parmi DECR1, CASP-3, SELP, JAM-A, la sous-unité A de PDFG, ITGB1BP2, Dkk-1, PECAM-1, ANG-1, SOD2, DAPP1, DCTN1, PAI, CD40-L, HB-EGF, HCLS1, IRAK4, STK4, SH2B3, NEMO, CD84, EIF4G1, PRDX1, TANK, CXCL1, SIRT2, ZBTB16, NF2, la sous-unité B de PDGF, SPRY2, HEXIM1, PPP1R9B, PRDX3, PAR-1, ICA1, CCL17, STAMPB, MGMT, SRPK2, la BML hydrolase, PLXNA4 et SORT1 ; ou dans lequel l'étape de détermination (a) comprend également la détermination du niveau quantitatif dans un second ensemble de biomarqueurs respectifs, dans lequel le niveau quantitatif du second ensemble de biomarqueurs est inférieur au niveau quantitatif du ou des mêmes biomarqueurs respectifs dans un échantillon normal, ce qui indique l'absence de réponse, le second ensemble de biomarqueurs est choisi parmi un ou plusieurs parmi DECR1, CD40-L, ITGB1BP2, CASP-3, la sous-unité A de PDGF, Dkk-1, SELP, SOD2, SORT1 et CD84.

12. Procédé selon la revendication 11, dans lequel le niveau quantitatif du ou de chaque biomarqueur est supérieur à une valeur seuil et indique l'absence de réponse, dans lequel la valeur seuil respective du ou de chaque biomarqueur est :
| **Biomarqueur** | **Valeur seuil** |
|---|---|
| DECR1 | 7,1320 |
| CASP-3 | 10,4740 |
| SELP | 10,5279 |
| Sous-unité A de PDGF | 5,5889 |
| ITGB1BP2 | 6,2200 |
| Dkk-1 | 10,4467 |
| SOD2 | 9,9025 |
| CD40-L | 7,4976 |
| CD84 | 6,0718 |
| SORT1 | 8,5585 |

13. Procédé selon la revendication 11, dans lequel le niveau quantitatif du ou de chaque biomarqueur est inférieur à une valeur seuil et indique l'absence de réponse, dans lequel la valeur seuil respective du ou de chaque biomarqueur est :
| **Biomarqueur** | **Valeur seuil** |
|---|---|
| DECR1 | 7,1320 |
| CASP-3 | 10,4740 |
| SELP | 10,5279 |
| Sous-unité A de PDGF | 5,5889 |
| ITGB1BP2 | 6,2200 |
| Dkk-1 | 10,4467 |
| SOD2 | 9,9025 |
| CD40-L | 7,4976 |
| CD84 | 6,0718 |
| SORT1 | 8,5585 |

14. Procédé selon l'une quelconque des revendications 1 à 6, dans lequel le niveau quantitatif du ou de chaque biomarqueur est compris entre deux valeurs seuils et indique une activité élevée de la maladie, dans lequel les valeurs seuils respectives du ou de chaque biomarqueur sont :
| **Biomarqueur** | **Valeur seuil** |
|---|---|
| DECR1 | 7,8984 - 11,6893 |
| CD40-L | 8,4728 - 12,7503 |
| ITGB1BP2 | 6,7802 - 11,4759 |
| CASP-3 | 11,4114 - 13,6768 |
| Sous-unité A de PDFG | 5,7334 - 8,4577 |
| Dkk-1 | 11,2034 - 12,7568 |
| SELP | 11,3715 - 12,6136 |
| SOD2 | 10,0471 - 10,5467 |
| SORT1 | 8,7990 - 9,7006 |
| CD84 | 6,1830 - 7,8683 |
ou dans lequel le niveau quantitatif du ou de chaque biomarqueur est compris entre deux valeurs seuils et indique une activité modérée de la maladie, dans lequel les valeurs seuils respectives du ou de chaque biomarqueur sont :
| **Biomarqueur** | **Valeurs seuil** |
|---|---|
| DECR1 | 5,6510 - 10,5039 |
| CD40-L | 6,1622 - 10,1159 |
| ITGB1BP2 | 3,1210 - 8,5301 |
| CASP-3 | 8,0945 - 12,2662 |
| Sous-unité A de PDFG | 3,9517 - 7,0510 |
| Dkk-1 | 9,1861 - 12,0037 |
| SELP | 9,2287 - 11,9099 |
| SOD2 | 9,5039 - 10,2607 |
| SORT1 | 7,5225 - 9,2595 |
| CD84 | 4,9694 - 6,8399 |
ou dans lequel le niveau quantitatif du ou de chaque biomarqueur est compris entre deux valeurs seuils et indique une activité faible de la maladie, dans lequel les valeurs seuils respectives du ou de chaque biomarqueur sont :
| **Biomarqueur** | **Valeurs seuil** |
|---|---|
| DECR1 | 6,6490 - 6,7309 |
| CD40-L | 6,5871 - 6,9401 |
| ITGB1BP2 | 4,1040 - 4,5746 |
| CASP-3 | 8,3368 - 8,7173 |
| Sous-unité | 3,8283 - 4,0907 |
| | |
|---|---|
| A de PDFG | |
| Dkk-1 | 9,6015 - 9,8410 |
| SELP | 8,2174 - 9,3414 |
| SOD2 | 9,6542 - 9,9702 |
| SORT1 | 7,7980 - 8,5272 |
| CD84 | 5,2689 - 5,5881 |
ou dans lequel le niveau quantitatif du ou de chaque biomarqueur est compris entre deux valeurs seuils et indique une rémission, dans lequel les valeurs seuils respectives du ou de chaque biomarqueur sont :
| **Biomarqueur** | **Valeur seuil** |
|---|---|
| DECR1 | 5,6064 - 6,9173 |
| CD40-L | 6,2510 - 7,3891 |
| ITGB1BP2 | 3,4508 - 5,5040 |
| CASP-3 | 7,9543 - 9,2353 |
| Sous-unité A de PDFG | 2,7648 - 4,9645 |
| Dkk-1 | 9,2598 - 9,9684 |
| SELP | 8,8272 - 9,9551 |
| SOD2 | 9,5195 - 9,7582 |
| SORT1 | 7,6829 - 8,4218 |
| CD84 | 4,6188 - 5,8750 |

15. Procédé selon l'une quelconque des revendications 1 à 14, dans lequel l'échantillon biologique est choisi parmi le sang total, le sérum, le plasma, l'urine, le liquide interstitiel, le liquide péritonéal, l'écouvillon cervical, les larmes, la salive, l'écouvillon buccal, la peau, le tissu cérébral et le liquide céphalo-rachidien.
